# EUROPEAN PATENT SPECIFICATION

(11) **EP 2 125 697 B1**
(45) Date of publication and mention of the grant of the patent: **28.09.2016**
(21) Application number: 07700593.2
(22) Date of filing: 15.01.2007
(51) Int. Cl.: C07C 219/10, A61K 31/215, A61P 35/00, A61P 17/00

(54) **POSITIVELY CHARGED WATER-SOLUBLE PRODRUGS OF RETINOIDS AND RETINOID-LIKE COMPOUNDS WITH VERY HIGH SKIN PENETRATION RATES**
POSITIV GELADENE WASSERLÖSLICHE PRODRUGS VON RETINOIDEN UND RETINOIDÄHNLICHEN VERBINDUNGEN MIT SEHR HOHEN HAUTPENETRATIONSRATEN
PROMÉDICAMENTS POSITIVEMENT CHARGÉS SOLUBLES DANS L'EAU DE RÉTINOÏDES ET DE COMPOSÉS SEMBLABLES À DES RÉTINOÏDES AYANT DES VITESSES DE PÉNÉTRATION DANS LA PEAU TRÈS ÉLEVÉES

(43) Date of publication of application: 02.12.2009
(73) Proprietor: Yu, Chongxi, Plainfield, Illinois 60585 (US)
(72) Inventor: Yu, Chongxi, Plainfield, Illinois 60585 (US)
(74) Representative: Finnegan Europe LLP
(86) International application number: PCT/IB2007/050122
(87) International publication number: WO 2008/087493

(56) References cited:
- EP-A1- 0 751 125
- WO-A1-00/47589
- WO-A1-2004/009538
- JP-A- 02 254 425
- US-A- 3 950 418
- US-A- 3 950 418
- US-A- 4 588 525
- DATABASE CAPLUS [Online] SHARMA P.K. ET AL.: 'Microwave assisted stereo selective synthesis of orgaomercurials from all-trans-retinoic acid', XP008111821 Retrieved from STN Database accession no. (2006:191480) & MAIN GROUP METAL CHEMISTRY vol. 28, no. 4, 2005, pages 208 - 212
- DATABASE CAPLUS [Online] AGAWA T. ET AL.: 'Stabilities of vitamin A urethans', XP008111828 Retrieved from STN Database accession no. 1956:42229 & KOGYO KAGAKU ZASSHI vol. 58, 1955, pages 686 - 688
- DATABASE CAPLUS [Online] KOMORI S. ET AL., XP008111827 Retrieved from STN Database accession no. (1955:74615) & BITAMIN vol. 8, 1955, pages 209 - 213

## Description

### Technical Field

The present invention relates to the preparations of positively charged and watersoluble pro-drugs of retinoids and retinoid-like compounds and their medicinal use in treating any retinoids and retinoid-like compounds-treatable conditions in humans or animals. More specifically, the present invention is to enable quick skin penetration of retinoids and retinoid-like compounds.

### Background Art

Retinoids are a class of compounds consisting of four isoprenoid units joined in a head to tail manner. The retinoids include all-trans-retinoic acid (tretinoin), cis-isomeric retinoic acids, e.g., 13-cis-retinoic acid (isotretinoin), 9-cis-retinoic acid (alitretinoin), vitamin A (retinol), 4-[1-(5,6,7,8-tetrahydro-3,5,5,8,8-pentamet hyl-2-naphthalenyl) ethenyl]benzoic acid (bexarotene, Targretin ®), retinal, retiferol, (E,E,E)-7-(2-n-propoxy-5,5,8,8-tetramethyl-5,6,7,8-tetrahydro-naphthalen-3-yl)-6-fluo ro-3-methylocta-2,4,6-trienoic acid, adapalene (6-[3-(1-adamantyl)-4-methoxyphenyl] - 2-naphthoic acid), acyclic retiniod [(2E, 4E, 6E, 10E)-3, 7, 11, 15-tetramethy-2,4,6,10,14-hexadecapentaenoic acid], ethyl(E,E,E)-7-(2-n-propoxy-5,5,8,8-tetramethyl-5,6,7,8-tetrahydro-naphthalen3-yl)-6-fluoro-3-methylocta-2,4,6-trienoate, and their derivatives, both natural and synthetic. Various synthetic retinoids and retinoid-like compounds having retinoid activity are described in various patents (U.S. Pat. Nos. 5,648,563; 5,648,385; 5,618,839; 5,559,248; 5,616,712; 5,616,597; 5,602,135; 5,599,819; 5,556,996; 5,534,516; 5,516,904; 5,498,755; 5,470,999; 5,468,879; 5,455,265; 5,451,605; 5,426,118; 5,407,937; 5,399,586; 5,399,561; 5,391,753 ). The retinoids are essential for many of life's processes including vision, reproduction, metabolism, differentiation, bone development, and pattern formation during embryogenesis. Vitamin A (retinol) and retinal are in chemical equilibrium in the body and have equivalent antixe-rophthalmic acitivity. Retinol combines with opsin, the rod pigment in the retina, to form rhodopsin, which is necessary for visual adaptation to darkness. Vitamin A deficiency is characterized by nyctalopia, keratomalacia, keratinization and drying of skin, lowered resistance to infection, retardation of growth, thickening of bone, d iminished production of cortical steroids, and fetal malformations (PDR Generics, 1996, second edition, Medical Economics, Montvale, New Jersey, pg 3094). Topical tretinoin (all-trans-retinoic acid) decreases cohesiveness of follicular epithelial cells with decreased microcomedo formation and stimulates mitotic activity increased turnover of follicular epithelial cells causing extrusion of the comedones. Tretinoin is indicated for topical application in the treatment of acne vulgaris, photoaging, hyper-pigmented macules (liver spot) and premature wrinkles, drug-induced photosensitivity, psoriasis, epidermal wound healing, xerophthalmia, keloids, hyperkeratotic skin disease (PDR Generics, 1996, second edition, Medical Economics, Montvale, New Jersey, pg 2981). Isotretinoin inhibits sebaceous gland function and keratinization. Isotretinoin is indicated for the treatment of severe recalcitrant cystic acne, basal cell carcinoma, cervical cancer, mycosis fungoides (cutaneous T-cell lymphoma), Darier's disease, lamellar ichthyosis, pityriasis rubra pilaris, herpes simplex infections, Grover's disease, lichen planus, refractory rosacea, keratosis palmaris et plantaris, leukoplakia, squamous cell skin cancer, and xeroderma pigmentosum. Alitretinoin (9-*cis*-retinoic acid) is a naturally-occurring endogenous retinoid that binds to and activates all known intracellular retinoid receptor subtypes (RAR, RAR, RAR, RXR, RXR , and RXR). Once activated these receptors function as transcription factors that regulate the expression of genes that control the process of cellular differentiation and proliferation in both normal and neoplastic cells. Alitretinoin inhibits the growth of Kaposi's sarcoma (KS) cells in vitro. Alitretinoin is used for the treatment of Kaposi's sarcoma and myelodysplastic syndromes. Retiferol derivatives are used for the treatment of hyperproliferative skin diseases such as psoriasis, basal cell carcinomas, disorders of keratinization and keratosis, neoplastic diseases, disorders of the sebaceous glands such as acne and seborrhoic dermatitis, the conditions associated with photodamage, the skin damaged through sun exposure, the effects of wrinkling, elastosis and premature ageing (Hilpert, et al., U.S. Pat. No. 6437142 ). Adapalene is used for the topical treatment of acne vulgaris. Acyclic retiniods are used for prevention of second primary tumors (Yasutoshi Muto, et al., the New England Journal of Medicine, 340, 1046 (1999)).(E,E,E)-7-(2-n-propoxy-5,5,8,8-tetramethyl-5,6,7,8-tetrahydro-naphthalen-3-yl )-6-fluoro-3-methylocta-2,4,6-trienoic acid may be used for the treatment of type 2 diabetes and other metabolic disorders (Deng T, et al., Biol. Pharm. Bull. 28(7), 1192, 2005). Targretin® oral formulation is used for the treatment of cutaneous T-cell lymphoma (CTCL), head and neck carcinoma, systemic Kaposi's sarcoma, lung cancer, ovarian cancer, prostate cancer, and renal cell cancer. Topical Targretin is used for the treatment of cutaneous T-cell lymphoma (CTCL).

One alternative method of administering drugs is topical delivery. Topical drug delivery has several advantages. This method helps to avoid inactivation of a drug caused by first pass metabolism in the liver and gastro-intestinal tract. It can provide local delivery of appropriate concentrations of a drug to the intended site of action without systemic exposure. Fishman (Fishman; Robert, U.S. Pat. No. 7,052,715) indicated that an additional problem associated with oral medications, is that the concentration levels which must be achieved in the bloodstream must be significant in order to effectively treat distal areas of pain or inflammation. These levels are often much higher than would be necessary if it were possible to accurately target the particular site of pain or injury. Yeager tried to use penetration enhancer to deliver PGE for the treatment of male erectile dysfunction (Yeager, James L. U.S. Pat. No. 6,693,135). Susan Milosovich, et al designed and prepared testosteronyl- 4-dimethylaminobutyrate.HCl (TSBH), which has a lipophilic portion and a tertiary amine group that exists in the protonated form at physiological pH. They found that the prodrug (TSBH) diffuses through human skin -60 times faster than does the drug (TS) itself [Susan Milosovich, et al., J. Pharm. ScL, 82, 227(1993)]. U.S. Pat. No. 3,950,418 discloses vitamin A acid amides.

### Disclosure of Invention

### Technical Problem

Retinoids and retinoid-like compounds are used to treat a variety of health conditions including acne, photoaging, psoriasis, ichthyosis, hair loss, and various cancers.

Unfortunately, retinoids and retinoid-like compounds are too lipophilic and practically insoluble in water. The membranes are bilayers, with the hydrophilic head groups facing outward into the aqueous regions on either side. Retinoids can enter the lipophilic membrane, but they will stay in the lipophilic layer as part of the membrane due to their similarities and cannot efficiently enter the cytosol on the inside of cell.

Owing to their high degree of unsaturation, retinoids are extremely sensitive to UV light, air, and oxidizing agents. After topical application of retinoids, they enter the membrane, but do not go into the inside of a cell. The sunlight, air, or oxidizing agent will induce retinoids' chemical reactions and cause skin redness, burning sensation, peeling, cracking, blistering, or itching. When they are taken orally, the first pass
metabolism, which refers to the chemical breakdown of compounds in the liver and gastro-intestinal tract, can destroy and inactivate them in a few minutes. Oral administration of retinoids creates unnecessary systemic exposure and causes many side effects.

### Technical Solution

The scope of the resent invention is defined by the appended claims. This invention relates to the design and preparation of novel positively charged pro-drugs of 9-cis-retinoic acid (alitretinoin) and their medicinal use. The prodrugs of 9-cis-retinoic acid (alitretinoin) have the general formula (1) 'Structure 1':

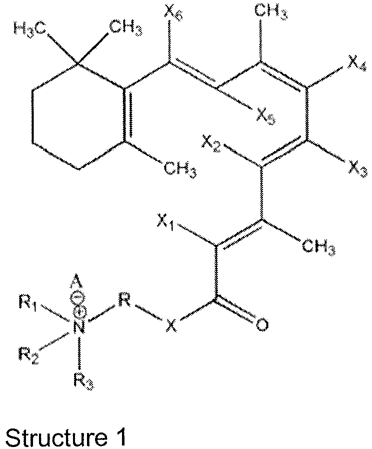

In structure 1, R represents a straight chain, -(CH₂)ₙ-, wherein n=1, 2, 3, 4, 5, 6, 7, 8, 9, or 10
R₁ and R₂ taken alone are same or different and are H, one of any alkyl, alkyloxyl, alkenyl or alkynyl residues having 1 to 12 carbon atoms, aryl or heteroaryl residues or taken together are -(CH₂) n -, wherein n=2, 3, 4, 5, 6, 7, 8, 9, or 10;
R₃ represents H;
X represents O, S, or NH;
X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy;
X₂ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy;
X₃ represents H, OH, Cl, Br, F, I, NO₂, NO, CN, SO₂R₅, COR₅, COOR₅, NR₆COR₅, SOR₅, SR₅, PO₃R₆R₆, C₁₋₆ alkyl, C₁₋₆ perfluoroalkyl, C₁₋₆ alkeny, C₁₋₆ alkyny, or C₁₋₆ alkyloxy;
X₄ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy;
X₅ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy;
X₆ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy;
R₅ represents H, OH, Cl, Br, F, I, NO₂, CN, SO₂R₆, COR₆, COOR₆, NR₆COR₇, SOR₆, SR₆, PO₃R₆R₆', SOR 5,' SR 5,' C₁₋₆ alkyl, C₁₋₆ alkyloxy, C₁₋₆ perfluoroalkyl, C₁₋₆ alkenyl, C₁₋₆ alkyny, or C₁₋₆ alkyl halide;
R₆ and R₆' taken alone are same or different and are H, Cl, Br, F, I, OH, NO₂, CN, SO₂R₅, COR₅, COOR₅, NR₇COR₅, SOR₅, SR₅, PO₃R₇R₇', C₁₋₆ alkyl, C₁₋₆ alkyloxy, C₁₋₆ alkenyl, C₁₋₆ alkyny, or alkyl halide or taken together are oxygen (=O) or -(CH₂)ₙ -, wherein n=2, 3, 4, 5, 6, 7, 8, 9, or 10
R₇ and R₇' taken alone are same or different and are H, Cl, Br, F, I, OH, NO₂, CN, SO₂R₅, COR₅, COOR₅, NR₇COR₅, SOR₅, SR₅, PO₃R₇R₇', C₁₋₆ alkyl, C₁₋₆ alkyloxy, C₁₋₆ alkenyl, C₁₋₆ alkyny, or alkyl halide or taken together are oxygen (=O) or -(CH₂)ₙ -, wherein n=2, 3, 4, 5, 6, 7, 8, 9, or 10

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. The membranes are bilayers, with the hy- drophilic head groups facing outward into the aqueous regions on either side. Very hy- drophilic drugs cannot pass the hydrophobic layer of a membrane and very hydrophobic drugs will stay in the hydrophobic layer as part of the membrane due to their similarities and cannot efficiently enter the cytosol on the inside.

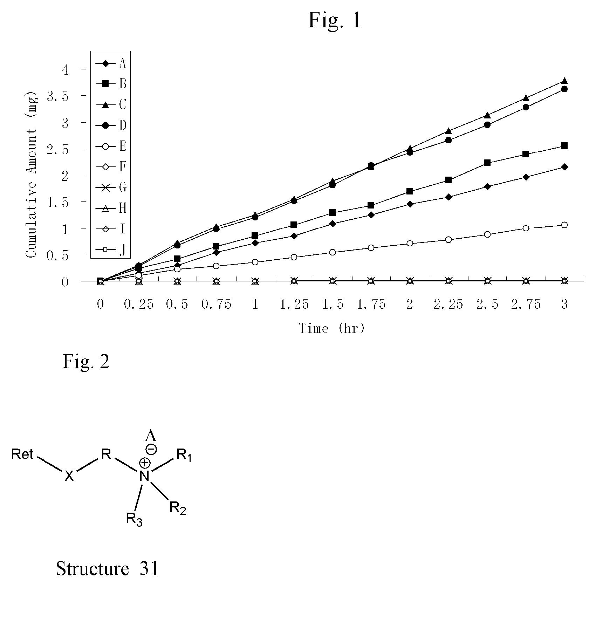

The goal of this invention is to make retinoinds and related compounds administrable transdermally (topical application) by increasing their solubility in the moisture available on the skin surface and their penetration rate through the membrane and skin barrier. These novel pro-drugs of retinoids and related compounds have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs in water. In many instances, the lowest or rate-limiting step in the sequence is the dissolution of the drug. Retinoids and related compounds have a very low solubility in the moisture available on the skin surface, and they will not pass across the barrier of skin in a molecular form efficiently. When they enter the membranes of the skin, they will stay there as part of the membrane due to their similarities and cannot efficiently enter the cytosol, a semi-liquid concentrated aqueous solution or suspension on the inside of the cell. When these new pro-drugs are administered transdermally in a dosage form such as a solution, spray, lotion, ointment, emulsion or gel, they will dissolve in the moisture available on the skin surface immediately. The positive charge on the amino groups of these pro-drugs will bond to the negative charge on the phosphate head group of a membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol. Due to the short stay outside the membranes of the skin, the pro-drugs will not cause burning, pain, itching, or swelling of the skin and the skin will be not sensitive to sun light. The penetration rates of these prodrugs through human skin were measured in vitro by using modified Franz cells, which were isolated from human skin tissue (360-400 µm thick) of the anterior and posterior thigh areas. The receiving fluid consisted of 2 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of either a 5% solution of some of the prodrugs of retinoids or a 5% suspension of retinoids in 0.2mL of the mixture of ethanol and pH 7.4-phosphate buffer (0.2M) (v/v, 70/30) are shown in Figure 1. Apparent flux values of 0.72 mg, 0.85 mg, 1.25 mg, 1.21 mg, 0.35 mg, 0.005 mg, 0.005 mg, 0.005 mg, 0.005 mg, and 0.001 mg/cm²/h were calculated for N,N-diethylaminoethyl 9-cis-retinoate.HBr, N,N-diethylaminoethyl 13-cis-retinoate.HBr, N,N-diethylaminoethyl all-trans-retinoate.HBr, retinyl N,N-dimethyl-2-aminoacetate.HCl, N,N-diethylaminoethyl 4-[1-(5,6,7,8-tetrahydro-3,5,5,8,8-pentamethyl-2-naphthalenyl)ethenyl]benzoate.HCl, 9-cis-retinoic acid (alitretinoin), 13-cis-retinoic acid (isotretinoin), all-trans-retinoic acid (tretinoin), vitamin A (retinol), and bexaroten (Targretin^{®}) respectively diffusing through human skin. The pro-drugs diffuse through human skin more than 350 times faster than do retinoids. The results suggest that the positive charge on the di-alkyaminoethyl group has a very important role in the passage of the drug across the membrane and skin barrier.

Irritative effect or discomfort in the skin of mice of the novel prodrugs was evaluated during a period of 1 week after the topical application of 0.1 ml of 1 % of the respective test drug in ethanol to the back of nude mice twice perday. None of any signs of irritative effect or discomfort was observed for N,N-diethylaminoethyl 9-cis-retinoate.HBr, N,N-diethylaminoethyl 13-cis-retinoate.HBr, N,N-diethylaminoethyl all-trans-retinoate.HBr, retinyl N,N-dimethyl-2-aminoacetate.HCl, N,N-diethylaminoethyl 4-[1-(5,6,7,8-tetrahydro-3,5,5,8,8-pentamethyl-2-naphthalenyl)ethenyl]benzoate.HCl.

A good prodrug should change back to the parent drug easily. In vitro plasma hydrolysis studies were carried out as the following. 10 mg of the prodrug was dissolved in 0.1 ml of 0.2M pH 7.4 phosphate buffer. 1 ml of human plasma, preheated to 37°C, was added into the mixture. The mixture was kept in a water bath at 37°C. At every 2 min intervals, 0.2 ml of samples were withdrawn and added to 0.4 ml of methanol to precipitate the plasma protein. The samples were centrifuged for 5 min and analyzed by HPLC. The half lives of hydrolysis are 10 min+/-1 min. for N,N-diethylaminoethyl 9-cis-retinoate.HBr, 8 min+/-2 min. for N,N-diethylaminoethyl 13-cis-retinoate.HBr, 9 min+/-1 min. for N,N-diethylaminoethyl all-trans-retinoate.HBr, 13 min+/-2 min. for retinyl N,N-dimethyl-2-aminoacetate.HCl, and 11 min.+/-2 min. for N,N-diethylaminoethyl 4-[1-(5,6,7,8-tetrahydro-3,5,5,8,8-pentamethyl-2-naphthalenyl)ethenyl]benzoate.HCl.

Targretin® (bexarotene) selectively activates a subclass of retinoid receptors called RXRs, which play an important role in several cellular activities. One of the most important of these activities is called programmed cell death, or 'apoptosis', a natural process by which the body rids itself of unwanted cells. Targretin® is being developed by Ligand in both topical and oral formulations. Topical Targretin is used for the treatment of cutaneous T-cell lymphoma (CTCL). In addition, Targretin oral formulation is used for the treatment of CTCL , head and neck carcinoma, systemic Kaposi's sarcoma, lung cancer, ovarian cancer, prostate cancer, and renal cell cancer. Alitretinoin (9-*cis*-retinoic acid) is a naturally-occurring endogenous retinoid that binds to and activates all known intracellular retinoid receptor subtypes (RARa, RARb , RARg, RXRa, RXRb and RXRg). Once activated these receptors function as transcription factors that regulate the expression of genes that control the process of cellular differentiation and proliferation in both normal and neoplastic cells. Al-itretinoid is used for treatment of Kaposi's Sarcoma, AI DS-Related Kaposi's Sarcoma, other skin cancer, breast cancer, and other cancers.

For evaluation of anti-tumor activity of these prodrugs, human breast cancer cells (BCAP-37, 3-4 mm³ of tumor tissue was used in each mouse) were subcutaneous xenografted into nude mice (BALB). After 1 days, 50 µl of 1 % N,N-diethylaminoethyl 9-cis-retinoate .HBr and N,N-diethylaminoethyl 4-[1-(5,6,7,8-tetrahydro-3,5,5,8,8-pentamethyl-2-naphthalenyl)ethenyl]benzoate.HCl in ethanol/0.2M pH 7.4 phosphate buffer (v/v, 70/30) was topically applied to the human breast cancer cells-implanted area (near the front leg) twice perday. After 28 days, the control group (n=7) demonstrated 100% incidence (the average tumor size was 13+2 mm x 12+2 cm), but none of tumor was seen in the test groups (n=7) that treated with N,N-diethylaminoethyl 9-cis-retinoate .HBr or N,N-diethylaminoethyl 4-[I-(5,6,7,8-tetrahydro-3,5,5,8,8-pentamethyl-2-naphthalenyl)ethenyl]benzoate.HCl. The most important thing is that mice that were given the drug did not show any discomfort or irritative effect. The average weight of the treated group is 25+2 grams and that of the control group is 23+3 grams. The results show that these prodrugs have very mild side effects.

In another experiment, human colon cancer cells (LS 174 J, 3-4 mm³ of tumor tissue was used in each mouse) were subcutaneous xenografted into nude mice (BALB). After 1 days, 50 µl of 1 % N,N-diethylaminoethyl 9-cis-retinoate.HBr and N,N-diethylaminoethyl 4-[I-(5,6,7,8-tetrahydro-3,5,5,8,8-pentamethyl-2-naphthalenyl)ethenyl]benzoate.HCl in ethanol/0.2M pH 7.4 phosphate buffer (v/v, 70/30) was topically applied to the human colon cancer cells-implanted area (near the front leg) twice perday. After 28 days, the control group (n=7) demonstrated 100% incidence (the average tumor size was 22+4 mm x 20+3 mm), but none of tumor was seen in the test groups (n=7) that treated with N,N-diethylaminoethyl 9-cis-retinoate .HBr or N,N-diethylaminoethyl 4-[I-(5,6,7,8-tetrahydro-3,5,5,8,8-pentamethyl-2-naphthalenyl)ethenyl]benzoate.HCl.

The retinoids are all commercially available. The compounds of the general formula (1) 'Structure 1' indicated above can be prepared from retinoic acids or related compounds, by reaction with compounds of the general formula ( 28 ) 'Structure 28 ' by using coupling reagents, such as N,N'-Dicyclohexylcarbodiimide, N, N'-Diisopropylcarbodiimide, O- (Benzotriazol- 1-yl)-N,N,N',N'-tetramethyluronium tetrafluoroborate, O- (Benzotriazol- 1 -yl)-N,N,N',N'- tetramethyluronium hexafluorophosphate, Benzotriazol-I-yl-oxy-tris(dimethylamino)phosphonium hexafluorophosphate, et al. 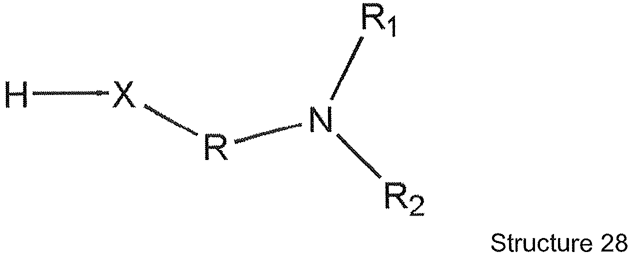 wherein, R₂ and X are defined as above.

When X represents 0, the compounds of the general formula (1) 'Structure 1 indicated above can be prepared from metal salts, organic base salts, or immobilized base salts of retinoic acids or related compounds, by reaction with compounds of the general formula (30) 'Structure 30'. 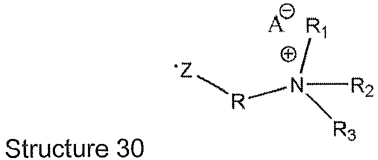 wherein, R, R₁, R₂, and X are defined as above.

### Advantageous Effects

These pro-drugs of retinoids and retinoid-like compounds in the present invention have a lipophilic portion and a hydrophilic portion (the amine groups that exist in the protonated form at physiological pH. The positively charged amino groups of these pro-drugs have two major advantages. First, it largely increases the solubility of the drugs in water; when these new pro-drugs are administered transdermally in a dosage form such as a solution, spray, lotion, ointment, emulsion or gel, they will mix with moisture on the skin, eye, genital area, mouth, nose, or other part of the body immediately. Second, the positive charge on the amino group of these pro-drugs will bond to the negative charge on the phosphate head group of the membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay on the skin, eye, genital area, mouth, nose, or other part of the body, the pro-drugs will not cause itching, burning or pain. Experiment results show that more than 90% of the pro-drugs were changed back to the parent drugs in a few minutes. The pro-drugs have a much better absorption rate and as
transdermal administration avoids the first pass metabolism , the pro-drugs will be stronger than retinoids and retinoid-like compounds at the same dosage. Another great benefit of transdermal administration of these pro-drugs is that administering medication, especially to children, will be much easier.

### Description of Drawings

Figure 1: Cumulative amounts of N,N-diethylaminoethyl 9-cis-retinoate.HBr (5% solution, A), N,N-diethylaminoethyl 13-cis-retinoate.HBr (5% solution, B), N,N-diethylarninoethyl all-trans-retinoate.HBr (5% solution, C), retinyl N,N-dimethyl-2-arninoacetate.HCl (5% solution, D), N,N-diethylaminoethyl 4-[I-(5,6,7,8-tetrahydro-3,5,5,8,8-pentamethyl-2-naphthalenyl)ethenyl]benzoate.HCl (5% solution, E), 9-cis-retinoic acid (5% suspention, F), 13-cis-retinoic acid (5% suspention, G ), all-trans-retinoic acid (5% suspention, H ), vitamin A (5% suspention, I ), and bexaroten (5% suspention, J ), crossing isolated human skin tissue in Franz cells (n=5). In each case, the vehicle was a ethanol/pH 7.4 phosphate buffer (0.2 M) (v/v, 70/30).
Figure 2: Structure 31, wherein, Ret represents retinoids and retinoid-like compounds; R represents a branched or straight chain, -(CH₂)ₙ-, wherein n=0, 1, 2, 3, 4, 5, 6, 7, 8, 9, 10 ..., aryl residues or heteroaryl residues; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, or 10 ; R₃ represents H, one of any alkyl, alkyloxy, alkenyl or alkynyl residues having 1 to 12 carbon atoms, or aryl and heteroaryl residues; R₄ represents H, one of any alkyl, alkyloxy, alkenyl or alkynyl residues having 1 to 12 carbon atoms, or aryl and heteroaryl residues; X represents O, S, or NH; A⁻ represents Cl⁻, Br⁻, F⁻, I⁻, AcO⁻, citrate, or any negative ions;

### Best Mode

### Preparation of N,N-diethylaminoethyl 9-cis-retinoate.HBr.

32.2 g (0.1 mol) of sodium 9-cis-retinoate was dissolved in 100 ml of acetonitrile.

26.1 g (0.1 mol) of 2-Bromo-N,N-diethylethylamine.HBr was added into the reaction mixture. The mixture was stirred for overnight at RT. The solvents were evaporated off. 200 ml of ethanol is added into the residue. The solid is removed by filtration. The solution is evaporated to dryness. 100 ml of ethyl acetate was added into the reaction mixture. Hexane (100 ml) was added. The solid product was collected by filtration. After drying, it yielded 36 g of the desired product (75%). Hygroscopic product. Elementary analysis: C₂₆H₄₂BrNO₃; MW: 480.52. Calculated % C: 64.99; H: 8.81; Br: 16.63; N: 2.91; O: 6.66; Found % C: 65.03; H: 8.80; Br: 16.60; N: 2.89; O: 6.68.

### Industrial Applicability

The pro-drugs of the general formula (1) 'Structure 1' are superior to retinoids and retinoid-like compounds. They can be used medicinally in treating any retinoids and retinoid-like compounds-treatable conditions in humans or animals. They may be used for the treatment of acne, acne scarring, psoriasis, ichthyosis, eczema, keratinization disorders, precancerous lesions, chemoprophylaxis, warts, sarcoidosis, treating photoaged skin, preventing photoaged skin, treating chronologically aged skin, hair loss, and various cancers.

## Claims

1. Compounds of Structure 1, 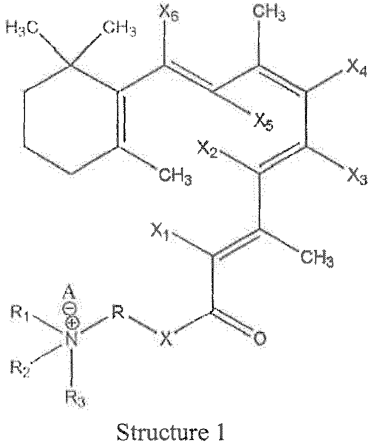
wherein R represents a straight chain -(CH₂)ₙ-, wherein n = 1, 2, 3, 4, 5, 6, 7, 8, 9, or 10;
R₁ and R₂ are the same or different, and are independently selected from the group consisting of H, alkyl, alkyloxyl, alkenyl and alkynyl residues having 1 to 12 carbon atoms, aryl and heteroaryl residues, or are taken together to be -(CH₂)ₙ-, wherein n = 2, 3, 4, 5, 6, 7, 8, 9, or 10;
R₃ is H;
X represents O, S, or NH;
X₁ is selected from the group consisting of H, OH, Cl, Br, F, I, NO₂, NO, CN, SO₂R₅, COR₅, COOR₅, NR₆ COR₅, SOR₅, SR₅, PO₃R₆R₆', C₁₋₆alkyl, C₁₋₆ perfluoroalkyl, C₁₋₆ alkenyl, C₁₋₆ alkynyl, and C₁₋₆ alkyloxy residues;
X₂ is selected from the group consisting of H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkenyl, C₁₋₆ alkynyl, and C₁₋₆ alkyloxy residues;
X₃ is selected from the group consisting of H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkenyl, C₁₋₆ alkynyl, and C₁₋₆alkyloxy residues;
X₄ is selected from the group consisting of H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkenyl, C₁₋₆ alkynyl, and C₁₋₆ alkyloxy residues;
X₅ is selected from the group consisting of H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkenyl, C₁₋₆ alkynyl, and C₁₋₆ alkyloxy residues;
X₆ is selected from the group consisting of H, OH, Cl, Br, F, I, NO₂, CN, SO₂R₅, COR₅, COOR₅, NR₆COR₅, SOR₅, SR₅, PO₃R₆R₆, C₁₋₆alkyl, C₁₋₆ perfluoroalkyl, C₁₋₆ alkenyl, C₁₋₆ alkynyl, and C₁₋₆ alkyloxy residues;
R₅ is selected from the group consisting of H, OH, Cl, Br, F, I, NO₂, CN, SO₂R₆, COR₆, COOR₆, NR₆COR₇, SOR₆, SR₆, PO₃R₆R₆', C₁₋₆ alkyl, C₁₋₆ alkyloxy, C₁-₆ perfluoroalkyl, C₁₋₆ alkenyl, C₁₋₆ alkynyl, and C₁₋₆ alkyl halide residues;
R₆ and R₆' are the same or different, and are independently selected from the group consisting of H, Cl, Br, F, I, OH, NO₂, CN, SO₂R₅, COR₅, COOR₅, NR₇COR₅, SOR₅, SR₅, PO₃R₇R₇', C₁₋₆ alkyl, C₁₋₆ alkyloxy, C₁₋₆ alkenyl, C₁₋₆ alkynyl, and alkyl halide residues, or are taken together to be oxo (=O) or -(CH₂)ₙ-, wherein n = 2, 3, 4, 5, 6, 7, 8, 9, or 10;
R₇ and R₇' are the same or different, and are independently selected from the group consisting of H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkenyl, C₁₋₆ alkynyl, and alkyl halide residues, or are taken together to be oxo (=O) or -(CH₂)ₙ-, wherein n = 2, 3, 4, 5, 6, 7, 8, 9, or 10; and
A⁻ represents any negative ions.

2. A compound according to claim 1, which is N,N-diethylaminoethyl 9-cis-retinoate· HA.

3. Processes for the preparation of a compound according to claim 1, wherein the compound is prepared from a retinoic acid, by reacting with a compound of Structure 28 in the presence of a coupling reagent selected from the group consisting of N,N'-dicyclohexylcarbodiimide, N,N'-diisopropylcarbodiimide, O-(benzotriazol-1-yl)-N,N,N',N'-tetramethyluronium tetrafluoroborate, O-(benzotriazol-1-yl)-N,N,N',N'-tetramethyluroniumhexafluorophosphate, and benzotriazol-1-yl-oxy-tris(dimethylamino)phosphonium hexafluorophosphate, 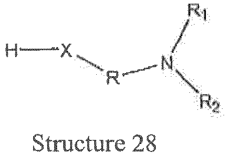 wherein R, R₁, R₂, and X are defined the same as in claim 1.

4. Processes for the preparation of a compound according to claim 1 or 2, wherein when X represents O, the compound is prepared from a metal salt, organic base salt, or immobilized base salt of a retinoic acid by reacting with a compound of Structure 30, 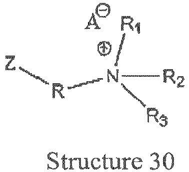 wherein R, R₁, R₂, R₃, and A⁻ are defined the same as in claim 1; and
Z represents halogen, or p-toluenesulphonyl;

5. The compound according to claim 1 or a composition comprising at least one compound according to claim 1 as an active ingredient for use in the treatment of a retinoids and retinoid-like compounds-treatable condition in a human or animal, wherein the compound or composition is administered orally or transdermally, wherein the retinoids and retinoid-like compounds-treatable condition is selected from the group consisting of acne, acne scarring, psoriasis, ichthyosis, eczema, keratinization disorders, precancerous lesions, chemoprophylaxis, warts, sarcoidosis, treating photoaged skin, preventing photoaged skin, treating chronologically aged skin, hair loss, cancer, Kaposi's Sarcoma, AIDS-Related Kaposi's Sarcoma, skin cancer, and breast cancer.

6. The compound according to claim 1 or a composition comprising at least one compound according to claim 1 as an active ingredient for use in the treatment of a retinoids and retinoid-like compounds-treatable condition in a human or animal, wherein the compound or composition is administered transdermally to any part of body, and wherein the compound or composition is in the form of a solution, spray, lotion, ointment, emulsion, or gel, wherein the retinoids and retinoid-like compounds-treatable condition is selected from the group consisting of acne, acne scarring, psoriasis, ichthyosis, eczema, keratinization disorders, precancerous lesions, chemoprophylaxis, warts, sarcoidosis, treating photoaged skin, preventing photoaged skin, treating chronologically aged skin, hair loss, cancer, Kaposi's Sarcoma, AIDS-Related Kaposi's Sarcoma, skin cancer, and breast cancer.

7. The compound according to claim 1 or a composition comprising at least one compound according to claim 1 as an active ingredient for use in the treatment of acne, acne scarring, psoriasis, ichthyosis, eczema, keratinization disorders, precancerous lesions, chemoprophylaxis, warts, sarcoidosis, photoaged skin, chronologically aged skin, hair loss, cancer, Kaposi's Sarcoma, AIDS-Related Kaposi's Sarcoma, skin cancer, and breast cancer, and in the prevention of photoaged skin in a human or animal, wherein the compound or composition is administered transdermally.

8. A transdermal therapeutic application system of a compound according to claim 1 or a composition comprising at least one compound according to claim 1 as an active ingredient for use in the treatment of a retinoids and retinoid-like compounds-treatable condition in a human or animal, wherein the retinoids and retinoid-like compounds-treatable condition is selected from the group consisting of acne, acne scarring, psoriasis, ichthyosis, eczema, keratinization disorders, precancerous lesions, chemoprophylaxis, warts, sarcoidosis, treating photoaged skin, preventing photoaged skin, treating chronologically aged skin, hair loss, cancer, Kaposi's Sarcoma, AIDS-Related Kaposi's Sarcoma, skin cancer, and breast cancer.

9. A transdermal therapeutic application system according to claim 8, **characterized in that** the system is a bandage or a patch comprising one active substance-containing matrix layer and an impermeable backing layer.

10. The transdermal therapeutic application system according to claim 8 or 9, **characterized by** having an active substance reservoir, which has a permeable bottom facing the skin.

11. The transdermal therapeutic application system according to one of claims 8 to 10, **characterized by** a controlling means controlling the rate of release, enabling the retinoid compounds to reach constantly optimal therapeutic blood levels to increase effectiveness and reduce the side effects of retinoid compounds.

## Patentansprüche

1. Verbindungen der Struktur 1, 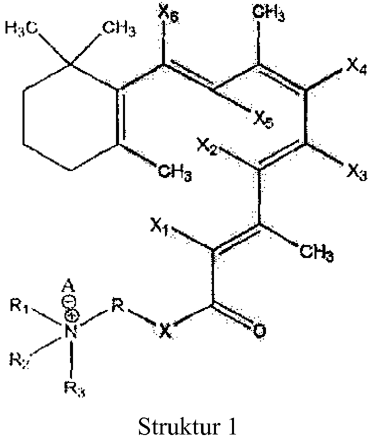
wobei R eine gerade Kette -(CH₂)ₙ- darstellt, wobei n = 1, 2, 3, 4, 5, 6, 7, 8, 9 oder 10;
R₁ und R₂ gleich oder verschieden sind und unabhängig ausgewählt sind aus der Gruppe bestehend aus H, Alkyl, Alkyloxyl, Alkenyl und Alkynylresten mit 1 bis 12 Kohlenstoffatomen, Aryl- und Heteroarylresten oder zusammen genommen werden, um -(CH₂)ₙ zu sein, wobei n = 2, 3, 4, 5, 6, 7, 8, 9 oder 10;
R₃ H ist;
X O, S oder NH darstellt;
X₁ ausgewählt ist aus der Gruppe bestehend aus H, OH, Cl, Br, F, I, NO₂, NO, CN, SO₂R₅, COR₅, COOR₅, NR₆ COR₅, SOR₅, SR₅, PO₃R₆R₆', C₁₋₆-Alkyl, C₁₋₆-Perfluoroalkyl, C₁₋₆-Alkenyl, C₁₋₆-Alkynyl und C₁₋₆-Alkyloxyresten;
X₂ ausgewählt ist aus der Gruppe bestehend aus H, OH, Cl, Br, F, I, NO₂, CN, SO₂R₅, COR₅, COOR₅, NR₆COR₅, SOR₅, SR₅, PO₃R₆R₆', C₁₋₆-Alkyl, C₁₋₆-Perfluoroalkyl, C₁₋₆-Alkenyl, C₁₋₆-Alkynyl und C₁₋₆-Alkyloxyresten;
X₃ ausgewählt ist aus der Gruppe bestehend aus H, OH, Cl, Br, F, I, NO₂, CN, SO₂R₅, COR₅, COOR₅, NR₆COR₅, SOR₅, SR₅, PO₃R₆R₆', C₁₋₆-Alkyl, C₁₋₆-Perfluoroalkyl, C₁₋₆-Alkenyl, C₁₋₆-Alkynyl und C₁₋₆-Alkyloxyresten;
X₄ ausgewählt ist aus der Gruppe bestehend aus H, OH, Cl, Br, F, I, NO₂, CN, SO₂R₅, COR₅, COOR₅, NR₆COR₅, SOR₅, SR₅, PO₃R₆R₆', C₁₋₆-Alkyl, C₁₋₆-Perfluoroalkyl, C₁₋₆-Alkenyl, C₁₋₆-Alkynyl und C₁₋₆-Alkyloxyresten;
X₅ ausgewählt ist aus der Gruppe bestehend aus H, OH, Cl, Br, F, I, NO₂, CN, SO₂R₅, COR₅, COOR₅, NR₆COR₅, SOR₅, SR₅, PO₃R₆R₆', C₁₋₆-Alkyl, C₁₋₆-Perfluoroalkyl, C₁₋₆-Alkenyl, C₁₋₆-Alkynyl und C₁₋₆-Alkyloxyresten;
X₆ ausgewählt ist aus der Gruppe bestehend aus H, OH, Cl, Br, F, I, NO₂, CN, SO₂R₅, COR₅, COOR₅, NR₆COR₅, SOR₅, SR₅, PO₃R₆R₆', C₁₋₆-Alkyl, C₁₋₆-Perfluoroalkyl, C₁₋₆-Alkenyl, C₁₋₆-Alkynyl und C₁₋₆-Alkyloxyresten;
R₅ ausgewählt ist aus der Gruppe bestehend aus H, OH, Cl, Br, F, I, NO₂, CN, SO₂R₆, COR₆, COOR₆, NR₆COR₇, SOR₆, SR₆, PO₃R₆R₆', C₁₋₆-Alkyl, C₁₋₆-Alkyloxy, C₁₋₆-Perfluoroalkyl, C₁₋₆-Alkenyl, C₁₋₆-Alkynyl und C₁₋₆-Alkylhalidresten;
R₆ und R₆' gleich oder verschieden sind und unabhängig ausgewählt sind aus der Gruppe bestehend aus H, Cl, Br, F, I, OH, NO₂, CN, SO₂R₅, COR₅, COOR₅, NR₇COR₅, SOR₅, SR₅, PO₃R₇R₇', C₁₋₆-Alkyl, C₁₋₆-Alkyloxy, C₁₋₆-Alkenyl, C₁₋₆-Alkynyl und Alkylhalidresten oder zusammen genommen werden, um Oxo (=O) oder -(CH₂)ₙ- zu sein, wobei n = 2, 3, 4, 5, 6, 7, 8, 9 oder 10;
R₇ und R₇' gleich oder verschieden sind und unabhängig ausgewählt sind aus der Gruppe bestehend aus H, Cl, Br, F, I, OH, NO₂, CN, SO₂R₅, COR₅, COOR₅, NR₆COR₅, SOR₅, SR₅, PO₃R₆R₆', C₁₋₆-Alkyl, C₁₋₆-Alkyloxy, C₁₋₆-Alkenyl, C₁₋₆-Alkynyl und Alkylhalidresten oder zusammen genommen werden, um Oxo (=O) oder -(CH₂)ₙ- zu sein, wobei n = 2, 3, 4, 5, 6, 7, 8, 9 oder 10; und
A⁻ negative Ionen darstellt.

2. Verbindung gemäß Anspruch 1, die N,N-Diethylaminoethyl-9-cis-retinoat-HA ist.

3. Verfahren zur Gewinnung einer Verbindung gemäß Anspruch 1, wobei die Verbindungen aus einer Retinsäure gewonnen wird, durch Reagieren mit einer Verbindung der Struktur 28 in Gegenwart eines Kopplungsreagens ausgewählt aus der Gruppe bestehend aus N,N'-Dicyclohexylcarbodiimid, N,N'-Diisopropylcarbodiimid, O-(Benzotriazol-1-yl)-N,N,N',N'-Tetramethyluronium-Tetrafluorborat, O-(Benzotriazol-1-yl)-N,N,N',N'- Tetramethyluronium-Hexafluorphosphat und Benzotriazol-1-yl-oxy-tris(dimethylamino)phosphonium-Hexafluorphosphat, 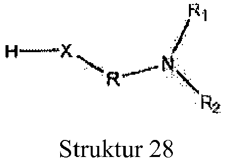 wobei R, R₁, R₂ und X gleich definiert sind wie in Anspruch 1.

4. Verfahren zur Gewinnung einer Verbindung gemäß Anspruch 1 oder 2, wobei, wenn X O darstellt, die Verbindung aus einem Metallsalz, organischen basischen Salz oder immobilisierten basischen Salz einer Retinsäure durch Reagieren mit einer Verbindung der Struktur 30 gewonnen wird, 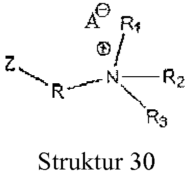 wobei R, R₁, R₂, R₃ und A⁻ gleich definiert sind wie in Anspruch 1; und Z Halogen oder p-Toluolsulfonyl darstellt;

5. Verbindung gemäß Anspruch 1 oder eine Zusammensetzung, die mindestens eine Verbindung gemäß Anspruch 1 als Wirkstoff zur Verwendung bei der Behandlung von einem mit Retinoid- und retinoid-artigen Verbindungen behandelbaren Zustand bei einem Patienten oder Tier beinhaltet, wobei die Verbindung oder Zusammensetzung oral oder transdermal verabreicht wird, wobei der mit Retinoid- und retinoid-artigen Verbindungen behandelbare Zustand ausgewählt ist aus der Gruppe bestehend aus Akne, Akne-Vernarbung, Psoriasis, Ichthyose, Ekzem, Verhornungsstörungen, präkanzerösen Läsionen, Chemoprophylaxe, Warzen, Sarkoidose, Behandeln lichtgealterter Haut, Verhüten lichtgealterter Haut, Behandeln chronologisch gealterter Haut, Haarverlust, Krebs, Kaposi-Sarkom, AIDS-bedingtes Kaposi-Sarkom, Hautkrebs und Brustkrebs.

6. Verbindung gemäß Anspruch 1 oder eine Zusammensetzung, die mindestens eine Verbindung gemäß Anspruch 1 als Wirkstoff zur Verwendung bei der Behandlung von einem mit Retinoid- und retinoid-artigen Verbindungen behandelbaren Zustand bei einem Patienten oder Tier beinhaltet, wobei die Verbindung oder Zusammensetzung transdermal an einen Körperteil verabreicht wird, und wobei die Verbindung oder Zusammensetzung in Form einer Lösung, eines Sprays, einer Lotion, einer Salbe, einer Emulsion oder eines Gels ist, wobei der mit Retinoid- und retinoid-artigen Verbindungen behandelbare Zustand ausgewählt ist aus der Gruppe bestehend aus Akne, Akne-Vernarbung, Psoriasis, Ichthyose, Ekzem, Verhornungsstörungen, präkanzerösen Läsionen, Chemoprophylaxe, Warzen, Sarkoidose, Behandeln lichtgealterter Haut, Verhüten lichtgealterter Haut, Behandeln chronologisch gealterter Haut, Haarverlust, Krebs, Kaposi-Sarkom, AIDS-bedingtes Kaposi-Sarkom, Hautkrebs und Brustkrebs.

7. Verbindung gemäß Anspruch 1 oder eine Zusammensetzung, die mindestens eine Verbindung gemäß Anspruch 1 als Wirkstoff zur Verwendung bei der Behandlung von Akne, Akne-Vernarbung, Psoriasis, Ichthyose, Ekzem, Verhornungsstörungen, präkanzerösen Läsionen, Chemoprophylaxe, Warzen, Sarkoidose, lichtgealterter Haut, chronologisch gealterter Haut, Haarverlust, Krebs, Kaposi-Sarkom, AIDS-bedingtes Kaposi-Sarkom, Hautkrebs und Brustkrebs, und bei der Verhütung von lichtgealterter Haut bei einem Patienten oder Tier beinhaltet, wobei die Verbindung oder Zusammensetzung transdermal verabreicht wird.

8. Transdermales, therapeutisches Anwendungssystem einer Verbindung gemäß Anspruch 1 oder einer Zusammensetzung, die mindestens eine Verbindung gemäß Anspruch 1 als Wirkstoff zur Verwendung bei der Behandlung von einem mit Retinoid- und retinoid-artigen Verbindungen behandelbaren Zustand bei einem Patienten oder Tier beinhaltet, wobei der mit Retinoid- und retinoid-artigen Verbindungen behandelbare Zustand ausgewählt ist aus der Gruppe bestehend aus Akne, Akne-Vernarbung, Psoriasis, Ichthyose, Ekzem, Verhornungsstörungen, präkanzerösen Läsionen, Chemoprophylaxe, Warzen, Sarkoidose, Behandeln lichtgealterter Haut, Verhüten lichtgealterter Haut, Behandeln chronologisch gealterter Haut, Haarverlust, Krebs, Kaposi-Sarkom, AIDS-bedingtes Kaposi-Sarkom, Hautkrebs und Brustkrebs.

9. Transdermales, therapeutisches Anwendungssystem gemäß Anspruch 8, **dadurch gekennzeichnet, dass** das System eine Bandage oder ein Pflaster ist, die eine Wirksubstanz enthaltende Matrixschicht und eine undurchlässige Trägerschicht beinhalten.

10. Transdermales, therapeutisches Anwendungssystem gemäß Anspruch 8 oder 9, **gekennzeichnet durch** das Aufweisen eines Wirksubstanzreservoirs, das einen durchlässigen Boden aufweist, der der Haut zugewandt ist.

11. Transdermales, therapeutisches Anwendungssystem gemäß einem der Ansprüche 8 bis 10, **gekennzeichnet durch** ein Steuerungsmittel zum Steuern der Rate der Freisetzung, wodurch es den Retinoid-Verbindungen ermöglicht wird, konstant optimale therapeutische Blutkonzentrationen zu erreichen, um die Wirksamkeit von Retinoid-Verbindungen zu vergrößern und ihre Nebenwirkungen zu reduzieren.

## Revendications

1. Composés de structure 1, 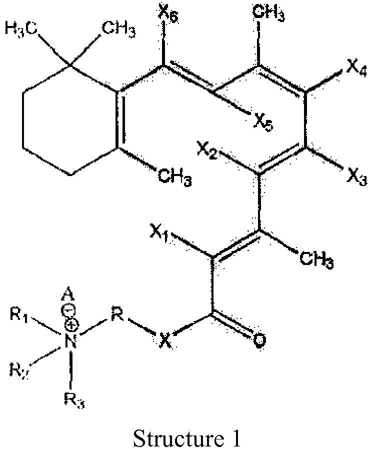
où R représente une chaîne droite -(CH₂)ₙ-, où n = 1, 2, 3, 4, 5, 6, 7, 8, 9 ou 10;
R₁ et R₂ sont identiques ou différents, et sont sélectionnés indépendamment dans le groupe composé de H, alkyle, alkyloxyle, alkényle et résidus d'alkényle ayant de 1 à 12 atomes de carbone, d'aryle et de résidus d'hétéroaryle, ou sont pris ensemble pour être -(CH₂)ₙ, où n = 2, 3, 4, 5, 6, 7, 8, 9 ou 10 ;
R₃ est H,
X représente O, S ou NH ;
X₁ est choisi dans le groupe composé de H, OH, Cl, Br, F, I, NO₂, NO, CN, SO₂R₅, COR₅, COOR₅, NR₆ COR₅, SOR₅, SR₅, PO₃R₆R₆', alkyle C₁₋₆, perfluoroalkyle C₁₋₆, alkényle C₁₋₆, alkynyle C₁₋₆ et résidus d'alkyloxy C₁₋₆ ;
X₂ est choisi dans le groupe composé de H, OH, Cl, Br, F, I, NO₂, CN, SO₂R₅, COR₅, COOR₅, NR₆COR₅, SOR₅, SR₅, PO₃R₆R₆', alkyle C₁₋₆, perfluoroalkyle C₁₋₆, alkényle C₁₋₆, alkynyle C₁₋₆ et résidus alkyloxy C₁₋₆ ;
X₃ est choisi dans le groupe composé de H, OH, Cl, Br, F, I, NO₂, CN, SO₂R₅, COR₅, COOR₅, NR₆COR₅, SOR₅, SR₅, PO₃R₆R₆', alkyle C₁₋₆, perfluoroalkyle C₁₋₆, alkényle C₁₋₆, alkynyle C₁₋₆ et résidus d'alkyloxy C₁₋₆;
X₄ est choisi dans le groupe composé de H, OH, Cl, Br, F, I, NO₂, CN, SO₂R₅, COR₅, COOR₅, NR₆COR₅, SOR₅, SR₅, PO₃R₆R₆', alkyle C₁₋₆, perfluoroalkyle C₁₋₆, alkényle C₁₋₆, alkynyle C₁₋₆ et résidus alkyloxy C₁₋₆ ;
X₅ est choisi dans le groupe composé de H, OH, Cl, Br, F, I, NO₂, CN, SO₂R₅, COR₅, COOR₅, NR₆COR₅, SOR₅, SR₅, PO₃R₆R₆', alkyle C₁₋₆, perfluoroalkyle C₁₋₆, alkényle C₁₋₆, alkynyle C₁₋₆ et résidus d'alkyloxy C₁₋₆;
X₆ est choisi dans le groupe composé de H, OH, Cl, Br, F, I, NO₂, CN, SO₂R₅, COR₅, COOR₅, NR₆COR₅, SOR₅, SR₅, PO₃R₆R₆', alkyle C₁₋₆, perfluoroalkyle C₁₋₆, alkényle C₁₋₆, alkynyle C₁₋₆ et résidus d'alkyloxy C₁₋₆;
R₅ est choisi dans le groupe composé de H, OH, Cl, Br, F, I, NO₂, CN, SO₂R₆, COR₆, COOR₆, NR₆COR₇, SOR₆, SR₆, PO₃R₆R₆', alkyle C₁₋₆, alkyloxy C₁₋₆, perfluoroalkyle C₁₋₆, alkényle C₁₋₆, alkynyle C₁₋₆ et résidus d'halogénure alkyle C₁₋₆,
R₆ et R₆' sont identiques ou différents, et sont choisis indépendamment dans le groupe de composé de H, Cl, Br, F, I, OH, NO₂, CN, SO₂R₅, COR₅, COOR₅, NR₇COR₅, SOR₅, SR₅, PO₃R₇R₇', alkyle C₁₋₆, alkyloxy C₁₋₆, alkényle C₁₋₆, alkynyle C₁₋₆ et résidus d'halogénure alkyle, ou sont pris ensemble pour être oxo (=O) ou -(CH₂)ₙ-, où n = 2, 3, 4, 5, 6, 7, 8, 9 ou 10 ;
R₇ et R₇' sont identiques ou différents, et sont choisis indépendamment dans le groupe de composé de H, Cl, Br, F, I, OH, NO₂, CN, SO₂R₅, COR₅, COOR₅, NR₆COR₅, SOR₅, SR₅, PO₃R₆R₆', alkyle C₁₋₆, alkyloxy C₁₋₆, alkényle C₁₋₆, alkynyle C₁₋₆ et résidus d'halogénure alkyle, ou sont pris ensemble pour être oxo (=O) ou -(CH₂)ₙ-, où n = 2, 3, 4, 5, 6, 7, 8, 9 ou 10 ; et
A⁻ représente des ions négatifs.

2. Composé selon la revendication 1, qui est le N,N-diéthylaminoéthyle 9-cis-retinoate-HA.

3. Processus de préparation d'un composé selon la revendication 1, où le composé est préparé à partir d'un acide rétinoïque, par réaction avec un composé de structure 28 en présence d'un réactif d'accouplement choisi dans le groupe composé de N,N'-dicyclohexylcarbodiimide, N,N'-diisopropylcarbodiimide, O-(benzotriazol-1-yle)-N,N,N',N'-tétraméthyluronium tétrafluoroborate, O-(benzotriazol-1-yle)-N,N,N',N'-tétraméthyluroniumhexafluorophosphate et benzotriazol-1-yl-oxy-tris(diméthylamino) phosphonium hexafluorophosphate, 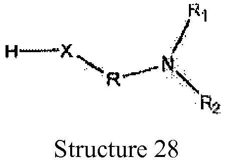 où R, R₁, R₂, et X sont définis de la même manière que dans la revendication 1.

4. Processus de préparation d'un composé selon la revendication 1 ou la revendication 2, où lorsque X représente O, le composé est préparé à partir d'un sel métallique, un sel base organique ou un sel de base immobilisé d'un acide rétinoïque par réaction avec un composé de structure 30, 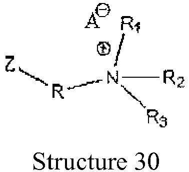 où R, R₁, R₂, R₃ et A⁻ sont définis de la même manière que dans la revendication 1 ; et Z représente un halogène ou un p-toluènesulphonyle.

5. Composé selon la revendication 1 ou composition comprenant au moins un composé selon la revendication 1 comme ingrédient actif pour utilisation dans le traitement de rétinoïdes et d'une pathologie pouvant être traitée par des composés de type rétinoïdes chez un être humain ou un animal, où le composé ou la composition sont administrés par voie orale ou transdermique, où les rétinoïdes et la pathologie pouvant être traité par des composés de type rétinoïdes sont choisis dans le groupe comprenant l'acné, les marques d'acné, le psoriasis, l'ichtyose, l'eczéma, les troubles de la kératinisation, les lésions précancéreuses, la chimioprophylaxie, les verrues, la sarcoïdose, le traitement de la peau photovieillie, la prévention de la peau photovieillie, le traitement de la peau chronologiquement âgée, la perte de cheveux, le cancer, le sarcome de Kaposi, le sarcome de Kaposi associé au SIDA, le cancer de la peau et le cancer du sein.

6. Composé selon la revendication 1 ou composition comprenant au moins un composé selon la revendication 1 comme ingrédient actif pour utilisation dans le traitement de rétinoïdes et d'une pathologie pouvant être traitée par des composés de type rétinoïdes chez un être humain ou un animal, où le composé ou la composition sont administrés par voie transdermique à toute partie de corps, et où le composé ou la composition sont sous forme d'une solution, d'une pulvérisation de lotion, d'onguent, d'émulsion ou de gel, où les rétinoïdes et la pathologie pouvant être traité par des composés de type rétinoïdes sont choisis dans le groupe comprenant l'acné, les marques d'acné, le psoriasis, l'ichtyose, l'eczéma, les troubles de la kératinisation, les lésions précancéreuses, la chimioprophylaxie, les verrues, la sarcoïdose, le traitement de la peau photovieillie, la prévention de la peau photovieillie, le traitement de la peau chronologiquement âgée, la perte de cheveux, le cancer, le sarcome de Kaposi, le sarcome de Kaposi associé au SIDA, le cancer de la peau et le cancer du sein.

7. Composé selon la revendication 1 ou composition comprenant au moins un composé selon la revendication 1 comme un ingrédient actif pour utilisation dans le traitement de l'acné, des marques d'acné, du psoriasis, de l'ichtyose, de l'eczéma, des troubles de la kératinisation, des lésions précancéreuses, de la chimioprophylaxie, des verrues, de la sarcoïdose, de la peau photovieillie, de la peau chronologiquement âgée, de la perte de cheveux, du cancer, du sarcome de Kaposi, du sarcome de Kaposi associé au SIDA, du cancer de la peau et du cancer du sein, et dans la prévention de la peau photoâgée chez un humain ou un animal, où le composé ou la composition sont administrés par voie transdermique.

8. Système d'application thérapeutique transdermique d'un composé selon la revendication 1 ou composition comprenant au moins un composé selon la revendication 1 comme ingrédient actif pour utilisation dans le traitement de rétinoïdes et d'une pathologie pouvant être traitée par des composés de type rétinoïdes chez un être humain ou un animal, où les rétinoïdes et la pathologie pouvant être traité par des composés de type rétinoïdes sont choisis dans le groupe comprenant l'acné, les marques d'acné, le psoriasis, l'ichtyose, l'eczéma, les troubles de la kératinisation, les lésions précancéreuses, la chimioprophylaxie, les verrues, la sarcoïdose, le traitement de la peau photovieillie, la prévention de la peau photovieillie, le traitement de la peau chronologiquement âgée, la perte de cheveux, le cancer, le sarcome de Kaposi, le sarcome de Kaposi associé au SIDA, le cancer de la peau et le cancer du sein.

9. Système d'application thérapeutique transdermique selon la revendication 8, **caractérisé en ce que** le système est un bandage ou un patch comprenant une couche matricielle contenant la substance active et une couche de renfort imperméable.

10. Système d'application thérapeutique transdermique selon la revendication 8 ou la revendication 9, **caractérisée par** l'utilisation d'un réservoir de substance active, qui a un fond perméable faisant face à la peau.

11. Système d'application thérapeutique transdermique selon l'une des revendications 8 à 10, **caractérisé par** un moyen de contrôle contrôlant la vitesse de libération, permettant aux composés de rétinoïde d'atteindre des concentrations sanguines thérapeutiques optimales de manière constante pour améliorer l'efficacité et réduire les effets secondaires des composés de rétinoïdes.
